# EUROPÄISCHE PATENTSCHRIFT

(11) **EP 3 612 239 B1**
(45) Veröffentlichungstag und Bekanntmachung des Hinweises auf die Patenterteilung: **03.04.2024**
(21) Anmeldenummer: 18709954.4
(22) Anmeldetag: 23.02.2018
(51) Int. Cl.: B65D 25/06, A61B 50/34, A61L 2/26

(54) **FLEXIBLES TRENNELEMENT FÜR SIEBKÖRBE**
FLEXIBLE DIVIDER FOR WIRE BASKETS
ÉLÉMENT DE SÉPARATION SOUPLE POUR PANIERS PERFORÉS

(30) Priorität: 21.04.2017 DE 102017206780
(43) Veröffentlichungstag der Anmeldung: 26.02.2020
(73) Patentinhaber: Aesculap AG, 78532 Tuttlingen (DE)
(72) Erfinder: BURMEISTER, Christoph, 78224 Singen (DE)
(74) Vertreter: Koller, Tobias Kilian
(86) Internationale Anmeldenummer: PCT/EP2018/054463
(87) Internationale Veröffentlichungsnummer: WO 2018/192703

(56) Entgegenhaltungen:
- EP-A2- 0 705 573
- DE-U1- 9 411 155
- US-A1- 2008 314 789

## Beschreibung

Die vorliegende Erfindung betrifft ein Trennelement für einen Sterilisationssiebkorb und insbesondere ein solches Trennelement, welches sich werkzeugfrei in einem Sterilisationssiebkorb positionieren und montieren lässt.

Im Stand der Technik sind zahlreiche Formen von Sterilisationssiebkörben bzw. Siebkörben bekannt. Diese werden dazu verwendet, um medizinische Instrumente und/oder Implantate darin zu lagern, diese Instrumente und Implantate zumindest teilweise in den Siebkörben zu reinigen (d.h. waschen) und den Siebkorb mit den Instrumenten und Implantaten dann in einen Sterilisationscontainer bzw. Sterilcontainer zu platzieren. In diesem Sterilcontainer wird der Siebkorb mit den darin befindlichen Instrumenten und Implantaten dann sterilisiert und gelagert, bis diese bei einer Operation benötigt werden. Bei der Operationsvorbereitung werden die Siebkörbe aus den Sterilcontainern entnommen und für den Operateur bereit gestellt. Teilweise verbleiben die Instrumente und Implantate während der Bereitstellung in den Siebkörben, teilweise werden diese aber auch zur Bereitstellung entnommen und beispielsweise auf einem Operationstuch bereit gelegt.

In der US 2008/0314789 A1 ist ein Lagerungselement für Sterilisationssiebkörbe offenbart, in welches z.B. Schaftinstrumente eingesetzt werden können, sodass diese einerseits nicht während des Waschvorgangs beschädigt werden und andererseits eine definierte Position während des Waschvorgangs haben, damit diese auch sauber werden. Dieses Lagerungselement ist im Boden von Siebkörben befestigbar und separiert den Innenraum des Sterilisationssiebkorbes zumindest teilweise. Aufgrund der Befestigung des Lagerungselements im Siebkorbboden und des mehrteiligen Aufbaus des Lagerungselements kann sich dieses Lagerungselement aber nicht über die komplette Breite des Siebkorbs erstrecken.

In der EP 0705573 A2 ist ein weiteres Lagerungselement gezeigt, welches an einem Gestell befestigbar ist, das wiederum in einen Sterilisationssiebkorb eingesetzt werden kann. Auch dieses Lagerungselement dient der Aufnahme uns Lagerung von Instrumenten und bewirkt eine teilweise Separierung unterschiedlicher Bereiche des Sterilisationssiebkorbes. Allerdings kann das Lagerungselement nicht frei positioniert werden, sondern kann ausschließlich an den vertikalen Streben des Gestells platziert werden.

Damit Instrumente und Implantate in einem Siebkorb zuverlässig gereinigt werden können, weisen die Seitenwände, der Boden und - soweit vorhanden - die Trennwände und ggf. Lagerungsstege Durchbrechungen auf, um den Durchtritt von Reinigungsfluid möglichst wenig zu behindern. In früheren Zeiten wurden Siebkörbe auch aus Metalldrahtgeflechten hergestellt. Da dies aber den Nachteil hat, dass sich an den Kreuzungspunkten der Metalldrähte leicht Schmutz ansammeln und einklemmen kann, ist man im medizinischen Bereich weitestgehend dazu über gegangen, Siebkörbe aus Metallblechen auszubilden, welche man mit einer Vielzahl von Durchbrechungen perforiert.

Siebkörbe gibt es in den unterschiedlichsten Größen und Formen. Teilweise werden Siebkörbe auch in anderen Siebkörben verstaut. Die in den Siebkörben gelagerten und aufbewahrten Teile können groß aber auch sehr klein sein. Damit die Teile nicht während der Reinigung durcheinander geraten, gibt es Siebkörbe mit Trennwänden. Die Aufteilung des Siebkorbes in mehrere Bereiche erleichtert auch die Identifikation von Teilen, das Aufnehmen dieser Teile aus dem Siebkorb oder die sichere Lagerung dieser Teile. Die Identifikation ist zum Beispiel erleichtert, wenn Pedikelschrauben mit unterschiedlicher aber ähnlicher Länge voneinander getrennt gelagert werden.

Bei diesen Siebkörben mit Trennwänden sind die Trennwände stets fest mit dem Siebkorb verbunden, zumeist indem sie angeschweißt oder angenietet sind. Dadurch ist es aber nicht möglich, dass ein Anwender die verschiedenen Bereiche bzw. Fächer seines Siebkorbes in der Größe und Form individuell an seine eigenen Bedürfnisse anpassen kann. Dies bedeutet, dass ein Hersteller entweder sehr viele verschiedene Siebkörbe mit unterschiedlicher Aufteilung herstellen und anbieten muss, oder dass dieses Bedürfnis des Anwenders nicht erfüllt werden kann. Das Herstellen vieler unterschiedlicher Siebkörbe in dann nur relativ geringer Stückzahl sowie das Vorhalten dieser Siebkörbe ist unverhältnismäßig teuer.

Die Aufgabe der vorliegenden Erfindung ist es daher, ein Trennelement für einen Sterilisationssiebkorb bereit zu stellen, welches es dem Anwender ermöglicht, seinen Sterilisationssiebkorb nach seinen Bedürfnissen zu unterteilen und in dem Sterilisationssiebkorb so Fächer individuell gewünschter Größe zu erzeugen. Eine weitere Aufgabe der vorliegenden Erfindung ist es, dass der Anwender dies werkzeugfrei vornehmen kann und dass er die Aufteilung zudem jederzeit an seine aktuellen Bedürfnisse anpassen kann.

Die Aufgabe der vorliegenden Erfindung wird durch ein Trennelement für einen Sterilisationssiebkorb gemäß Anspruch 1 gelöst. Vorteilhafte Ausgestaltungen solcher Trennelemente sind Gegenstand der abhängigen Ansprüche.

Gemäß einer ersten Ausführungsform der vorliegenden Erfindung ist ein Trennelement für einen Sterilisationssiebkorb zur Sterilisation von medizinischen Instrumenten vorgesehen, welches zwei laterale Wandabschnitte und einen dazwischen liegenden zentralen Wandabschnitt aufweist. Das Trennelement ist im Wesentlichen flächig ausgebildet, wobei es auch möglich ist, dass das Trennelement gewellt oder im Zick-Zack ausgebildet ist. Flächig bezieht sich in hierbei lediglich darauf, dass durch das Trennelement eine Trennwand ausgebildet wird, welche dadurch gekennzeichnet ist, dass sie in zwei Raumrichtungen eine deutlich größere Erstreckung aufweist als in die dritte Raumrichtung. Der zentrale Wandabschnitt ist mit einer Vielzahl von Durchbrechungen ausgebildet, das heißt, er ist mit einer flächigen Perforierung ausgebildet. Einer der beiden lateralen Wandabschnitte weist zudem eine schlitzförmige Aussparung auf. Dadurch ist in diesem lateralen Wandabschnitt der laterale Bereich als federelastischer Abschnitt ausgebildet. Dies bedeutet, dass dieser federelastische Bereich, welcher den einen lateralen Rand des Trennelements darstellt, elastisch zu dem zentralen Bereich hin verformt werden kann, sodass sich die maximale laterale Erstreckung des Trennelements verringert.

Der federelastischer Abschnitt weist zudem eine Anzahl von Vorsprüngen auf, welche sich zumindest in lateraler Richtung erstrecken. Zumindest in lateraler Richtung erstrecken bedeutet, dass sich diese Vorsprünge abschnittsweise oder ganz auch in eine Querrichtung erstrecken können, also beispielsweise schräg, relevant ist hier aber die Erstreckung in lateraler Richtung, sodass diese Vorsprünge in Aussparungen eingreifen können, welche in einem Siebkorb vorgesehen sind, in welchen das Trennelement eingesetzt und montiert werden soll. Außerdem ist die Anzahl von Vorsprüngen daran angepasst, in einer Wand eines Sterilisationssiebkorbes und/oder in einem anderen Trennelement gemäß dieser Erfindung verankert zu werden. Diese Vorsprünge können sich auch komplett in der Ebene erstrecken, welche durch die Trennwand des Trennelements definiert ist. Der Schlitz ist zudem breit genug, um eine ausreichende elastische Verformung des federelastischen Bereichs zuzulassen, sodass die an diesem federelastischen Bereich vorgesehenen Vorsprünge außer Eingriff mit den Perforationen des Wandabschnitts gebracht werden können, in den die zur Montage des Trennelements einrücken.

Der andere laterale Wandabschnitt weist ebenfalls eine Anzahl von Vorsprüngen auf, welche sich zumindest teilweise in lateraler Richtung erstrecken. Auch diese Vorsprünge sind dafür vorgesehen, in Aussparungen einzugreifen, welche in einer Wand eines Siebkorbs oder einem anderen in dem Siebkorb angeordneten erfindungsgemäßen Trennelement vorgesehen sind.

Vorzugsweise sind diese letztgenannten Vorsprünge so ausgebildet, dass sie sich auch zumindest teilweise in eine Richtung erstrecken, welche senkrecht zu der Erstreckungsebene des zentralen Wandabschnitts angeordnet ist. Diese Erstreckungsebene ist sozusagen die Zwischenwandfläche bzw. Trennwandfläche, welche in dem Siebkorb durch das Trennelement gebildet wird.

Gemäß einer vorteilhaften Ausführungsform des ersten Aspekts der vorliegenden Erfindung sind die Vorsprünge des anderen lateralen Wandbereichs im Wesentlichen L-förmig ausgebildet. Sie erstrecken sich von dem anderen lateralen Wandabschnitt zunächst in lateraler Richtung und im weiteren Verlauf in einer Richtung, welche senkrecht zur Fläche des zentralen Wandabschnitts verläuft.

Gemäß einer zweiten Ausführungsform der vorliegenden Erfindung ist ein Trennelement für einen Sterilisationssiebkorb zur Sterilisation von medizinischen Instrumenten vorgesehen, welches zwei laterale Wandabschnitte und einen dazwischen liegenden zentralen Wandabschnitt aufweist. Das Trennelement ist im Wesentlichen flächig ausgebildet, wobei es auch hier möglich ist, dass das Trennelement gewellt oder im Zick-Zack ausgebildet ist. Der zentrale Wandabschnitt ist auch hier mit einer Vielzahl von Durchbrechungen ausgebildet. Beide lateralen Wandabschnitte weisen je eine schlitzförmige Aussparung auf, sodass jeweils der durch die schlitzförmige Aussparung separierte, laterale Bereich jedes lateralen Wandabschnitts als federelastischer Abschnitt ausgebildet ist. Bei dieser Ausführungsform sind demnach an beiden lateralen Seiten des Trennelements ein federelastischer Abschnitt vorgesehen, wie er bereits zur ersten Ausführungsform beschrieben ist. Beide federelastischen Abschnitte weisen jeweils eine Anzahl von Vorsprüngen auf, welche sich zumindest in lateraler Richtung erstrecken.

Gemäß einer vorteilhaften Ausführung des zweiten Aspekts der vorliegenden Erfindung ist das Trennelement spiegelsymmetrisch ausgebildet. Dies erleichtert das richtige Einsetzen des Trennelements in einen Siebkorb, da nicht auf die korrekte Ausrichtung rechts-links geachtet werden muss. Dies bedeutet, dass es vollkommen egal ist, mit welcher Seite des Trennelements man es zuerst in einen Siebkorb einsetzt und mittels der Vorsprünge in der einen Wand verankert und anschließend die andere Seite des Trennelements mit den Vorsprüngen in der gegenüberliegenden Wand verankert.

Gemäß einer anderen vorteilhaften Ausführung des zweiten Aspekts ist das Trennelement rotationssymmetrisch ausgebildet. Dies erleichtert das richtige Einsetzen des Trennelements in einem Siebkorb, da nicht auf eine korrekte Ausrichtung oben-unten geachtet werden muss. Bei dieser Ausführung gibt es kein oben und unten an dem Trennelement mehr, sondern es befinden sich einige Vorsprünge an einer Seite im oberen Bereich und andere Vorsprünge an der Anderen Seite im unteren Bereich des Trennelements. So kann man zum Beispiel unabhängig von der Ausrichtung des Trennelements dieses in einen Siebkorb einsetzen, indem man es zunächst mit den Vorsprüngen im unteren Bereich in einer Wand eines Siebkorbs verankert und anschließend mit den Vorsprüngen an der anderen Seite, welche sich demnach im oberen Bereich befinden, in einer gegenüberliegenden Wand verankert. Selbstverständlich geht es auch in umgekehrter Reihenfolge, aber der federelastische Abschnitt ist wohl leichter elastisch zu verformen, wenn er im oberen Bereich des Trennelements gegriffen werden kann und sich die Vorsprünge auch im oberen Bereich des Trennelements befinden. Die vorstehend beschriebene Vorgehensweise ist vermutlich die einfachere Art und Weise, ein dementsprechend erfindungsgemäßes Trennelement in einem Siebkorb zu montieren.

Gemäß einer vorteilhaften Ausführung beider Aspekte der vorliegenden Erfindung weist jeder laterale Wandbereich wenigstens zwei Vorsprünge auf. Auf diese Weise ist einfach sicher gestellt, dass sich ein in einem Siebkorb montiertes Trennelement nicht mehr durch Querkräfte kippen kann, da die beiden Vorsprünge einem Kippmoment entgegen wirken. Hierzu ist es vorteilhaft, wenn die beiden Vorsprünge je Seite einen gewissen Abstand voneinander aufweisen.

Gemäß noch einer vorteilhaften Ausführung beider Aspekte der vorliegenden Erfindung ist an einem federelastischen Abschnitt ein Eingriffsabschnitt ausgebildet. Dieser erleichtert sowohl das elastische Verformen der federelastischen Abschnitte, um somit die laterale Ausdehnung des Trennelements vorübergehend zu verringern, um so die an dem federelastischen Abschnitt vorgesehenen Vorsprünge zunächst an Löchern eines Siebkorbes vorbei zu führen, um sie und das gesamte Trennelement zu positionieren und anschließend durch entspannen des federelastischen Abschnitts in die gewünschten Löcher des Siebkorbes einzuführen. Gleichzeitig gibt ein solcher Eingriffsabschnitt einen intuitiven Benutzungshinweis an den Nutzer, nämlich dass er das Trennelement an dieser Stelle greifen und elastisch verformen soll.

Vorzugsweise ist der Eingriffsabschnitt zwischen zwei Vorsprüngen ausgebildet. Dadurch ist gleichzeitig auch sicher gestellt, dass diese beiden Vorsprünge einen ausreichenden Abstand voneinander haben, um Kippmomente aufzunehmen. Zudem kann so einfach sicher gestellt werden, dass der Nutzer intuitiv eine ausreichende elastische Verschiebung der Vorsprünge zur Mitte des Trennelements hin vornimmt, sodass er nicht versehentlich mit den Vorsprüngen in Löchern des Siebkorbes hängen bleibt, in denen er das Trennelement nicht verankern möchte.

Weiter vorzugsweise ist der Eingriffsabschnitt als laterale Aussparung ausgebildet, beispielsweise als bogenförmige Aussparung. Auf diese Weise kann ein sehr einfacher Eingriffsabschnitt ausgebildet werden, welcher gleichzeitig intuitiv einen Hinweis an den Nutzer gibt, den federelastischen Abschnitt in die Richtung zu verformen, in welche die laterale Aussparung zeigt.

Besonders vorteilhaft ist das Trennelement aus Metall ausgebildet, insbesondere aus einem Metallblech. Metall eignet sich hervorragend für Sterilisationszubehör, sofern es ein nicht rostendes Metall wie beispielsweise ein Edelstahl oder Aluminium oder eine Legierung von diesen ist. Ist das Trennelement aus einem Metallblech gefertigt, kann man alle Strukturen in einem Arbeitsschritt z.B. mittels Stanzen, Laserschneiden, Wasserstrahlschneiden, Drahterodieren oder dergleichen aus dem Blech ausformen. Ggf. müssen dann lediglich noch einige Vorsprünge in die L-Form gebracht werden, wenn es sich um ein Trennelement gemäß dem ersten Aspekt der Erfindung handelt. Andernfalls ist nicht einmal mehr dieser Arbeitsschritt erforderlich. Dies ermöglicht eine sehr einfache und vollautomatische Fertigung.

Weitere Vorteile und Merkmale der Erfindung sind dem Fachmann aus den beigefügten Figuren und der detaillierten Beschreibung der Ausführungsbeispiele ersichtlich. Hierbei zeigt
- Fig. 1: eine Ansicht eines Trennelements gemäß einem ersten Ausführungsbeispiel der vorliegenden Erfindung;
- Fig. 2: eine perspektivische Ansicht des Trennelements der Fig. 1;
- Fig. 3A und 3B: den Anfang eines Einbaus eines Trennelements der Fig. 1 in einen Siebkorb;
- Fig. 4A und 4B: den Fortgang eines Einbaus eines Trennelements der Fig. 1 in einen Siebkorb;
- Fig. 5: einen Siebkorb mit mehreren Trennelementen entsprechend der Fig. 1;
- Fig. 6: eine Ansicht eines zweiten Ausführungsbeispiels der vorliegenden Erfindung;
- Fig. 7: eine Ansicht eines dritten Ausführungsbeispiels der vorliegenden Erfindung;
- Fig. 8: eine Ansicht eines vierten Ausführungsbeispiels der vorliegenden Erfindung;
- Fig. 9: eine perspektivische Ansicht des Trennelements der Fig. 8;
- Fig. 10A und 10B: den Anfang eines Einbaus eines Trennelements der Fig. 8 in einen Siebkorb;
- Fig. 11A und 11B: den Fortgang eines Einbaus eines Trennelements der Fig. 8 in einen Siebkorb; und

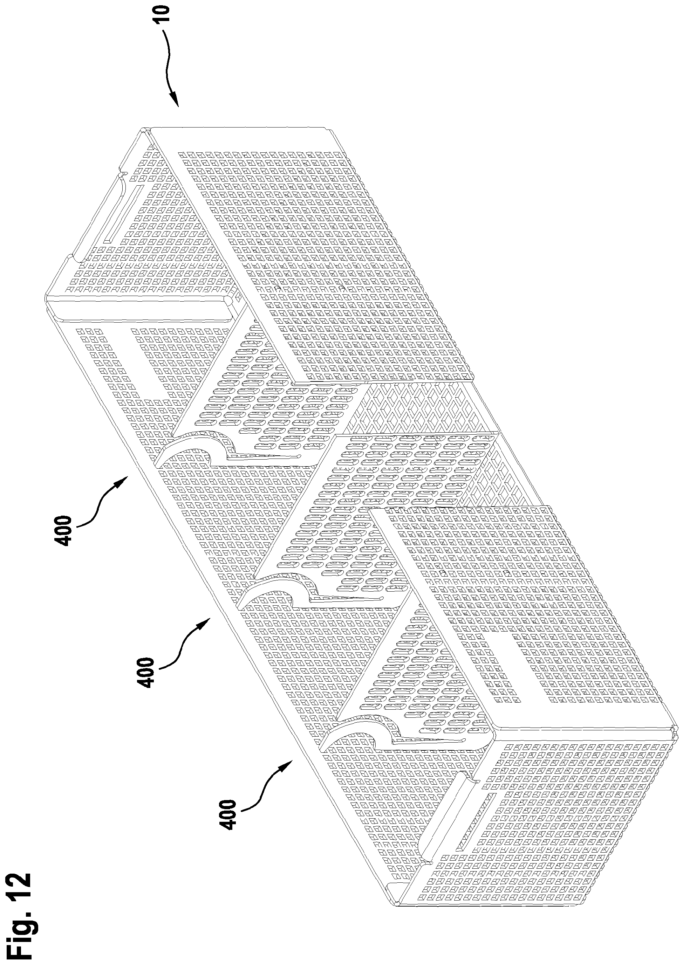

- Fig. 12: einen Siebkorb mit mehreren Trennelementen entsprechend der Fig. 8.

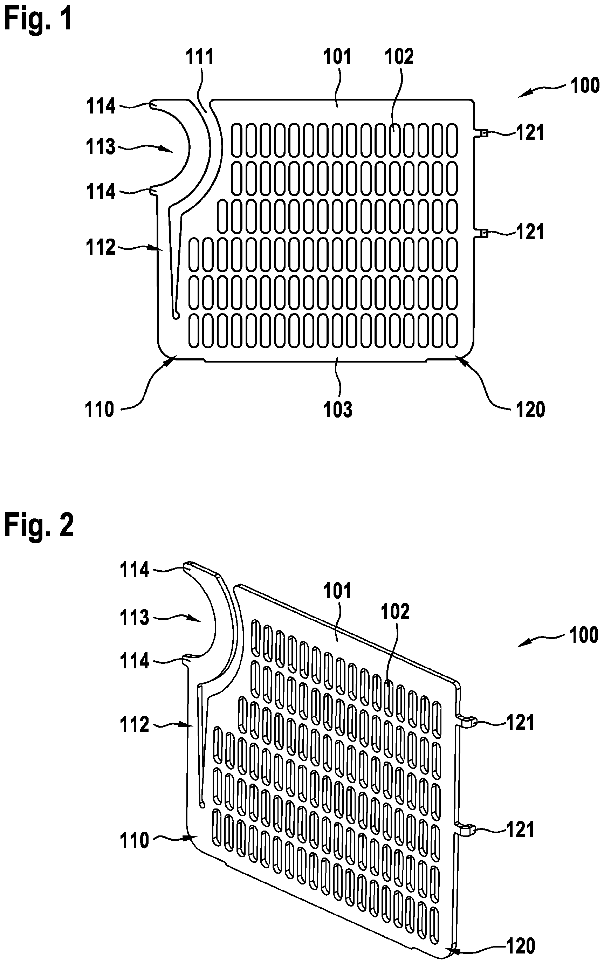

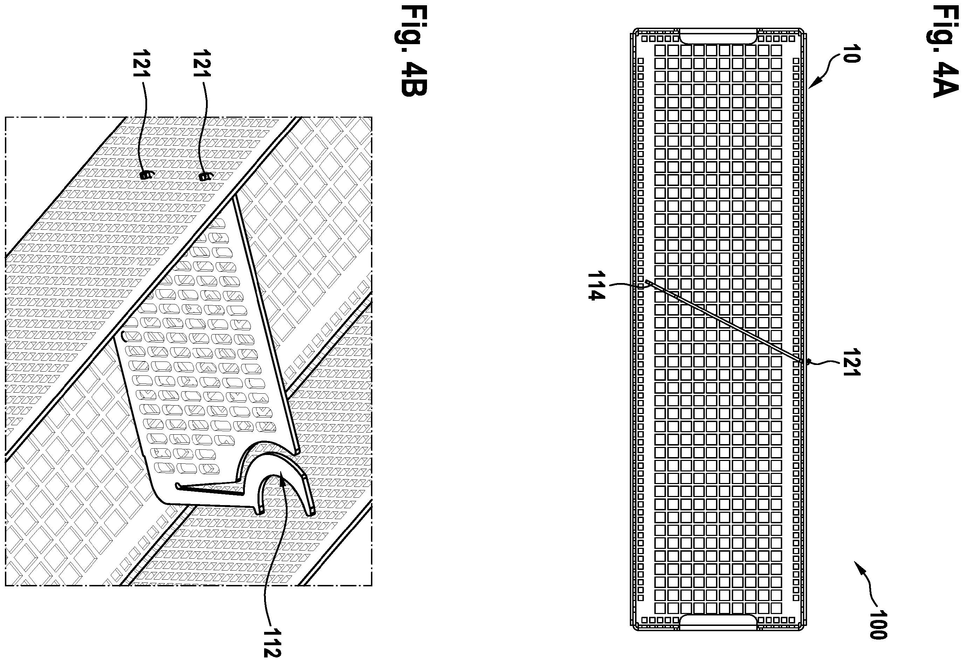

Das erste Ausführungsbeispiel der vorliegenden Erfindung ist unter Bezugnahme auf die Fig. 1 bis 5 im Detail beschrieben.

In Fig. 1 ist ein Trennelement 100 gemäß dem ersten Ausführungsbeispiel in einer Ansicht gezeigt. Das Trennelement 100 ist aus einem 2 mm dicken Edelstahlblech gefertigt und weist einen zentralen Wandabschnitt 101, einen ersten lateralen Wandabschnitt 110 und einen zweiten lateralen Wandabschnitt 120 auf. In dem ersten lateralen Wandabschnitt 110 ist eine schlitzförmige Aussparung 111 ausgebildet, welche einen federelastischen Abschnitt 112 von dem zentralen Wandabschnitt trennt und dem federelastischen Abschnitt 112 den Raum gibt, zu dem zentralen Wandabschnitt 101 hin verformt zu werden. An dem federelastischen Abschnitt 112 sind zwei laterale Vorsprünge 114 ausgebildet, zwischen welchen ein Eingriffsabschnitt 113 ausgebildet ist. Der Eingriffsabschnitt 113 ist als laterale Aussparung ausgebildet, welche die Form eines Kreisbogens aufweist. Der Kreisboden zeigt zu dem zentralen Wandabschnitt 101 hin und zeigt dem Nutzer dadurch die Richtung, in welche er den federelastischen Abschnitt verformen muss, um das Trennelement 100 in einem Siebkorb zu montieren. Die Vorsprünge 114 sind bei diesem Ausführungsbeispiel genau so lang, wie die Wand des Siebkorbs dick ist, in welchen das Trennelement eingesetzt werden soll. In dem zweiten lateralen Wandabschnitt sind zwei Vorsprünge 121 ausgebildet. Wie dies aus der Fig. 2 ersichtlich ist, sind diese beiden Vorsprünge 121 L-förmig ausgebildet. Diese L-förmigen Vorsprünge erstrecken sich zunächst in lateraler Richtung in etwa so weit vom Rand des Trennelements 100 vor, wie die Wand des Siebkorbs dick ist, in den das Trennelement eingesetzt werden soll. Anschließend erstrecken sich die Vorsprünge in etwa so weit in eine Richtung senkrecht zu der Fläche des Trennelements - in der Fig. 1 nach hinten - wie zwei Löcher in der Wand des Siebkorbs beabstandet sind, in den das Trennelement eingesetzt werden soll. Der zentrale Wandabschnitt 101 weist zahlreiche Durchbrechungen bzw. Löcher auf, welche in Reihen und Spalten angeordnet sind und somit eine gitterartige Struktur bilden. Bei diesem Ausführungsbeispiel haben die Löcher eine ovale Form, wobei die Erfindung darauf nicht beschränkt ist. Zudem weist der zentrale Wandabschnitt 101 einen Anlagevorsprung 103 auf, der in Anlage an den Boden des Siebkorbs gebracht wird, wenn das Trennelement 100 in einen Siebkorb eingesetzt wird. Dieser Anlagevorsprung 103 erleichtert die vertikale Ausrichtung des Trennelements zu dem Siebkorb beim Einsetzen. Die Wandabschnitte 101, 110 und 120 gehen ineinander über und sind nicht zwangsläufig scharf gegeneinander abgegrenzt.

In diesem Beispiel weist die schlitzförmige Aussparung 111 an ihrer Wurzel eine Ausrundung auf, um ein Kerben des Trennelements in diesem Bereich aufgrund eine Vielzahl von elastischen Verformungen des federelastischen Abschnitts 112 zu verhindern. Das heißt, die Ausrundung dient dazu, Spannungsspitzen in dem Trennelement 100 im verformten Zustand des federelastischen Abschnitts 112 zu verringern.

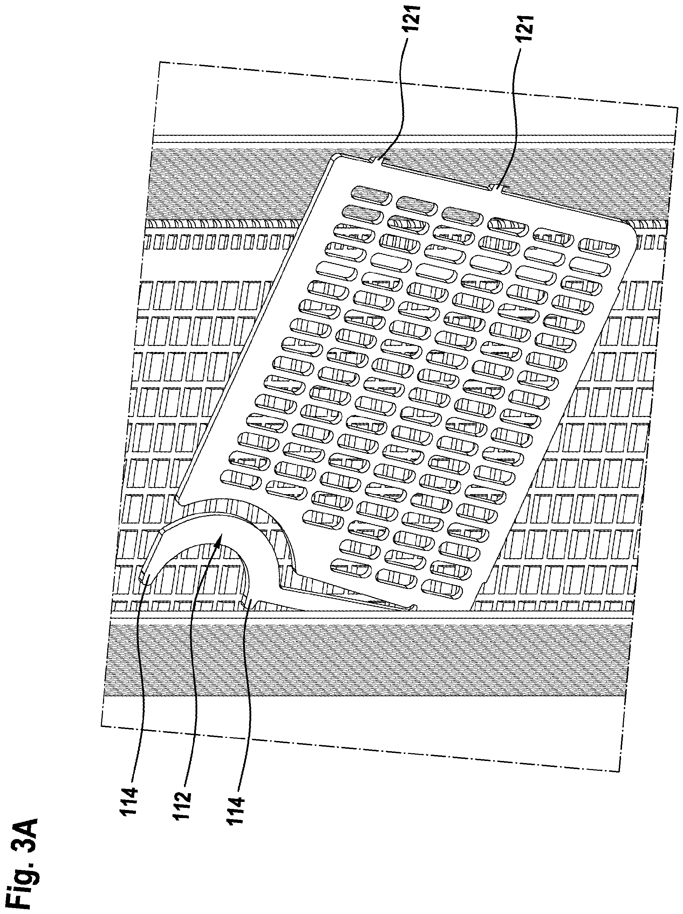

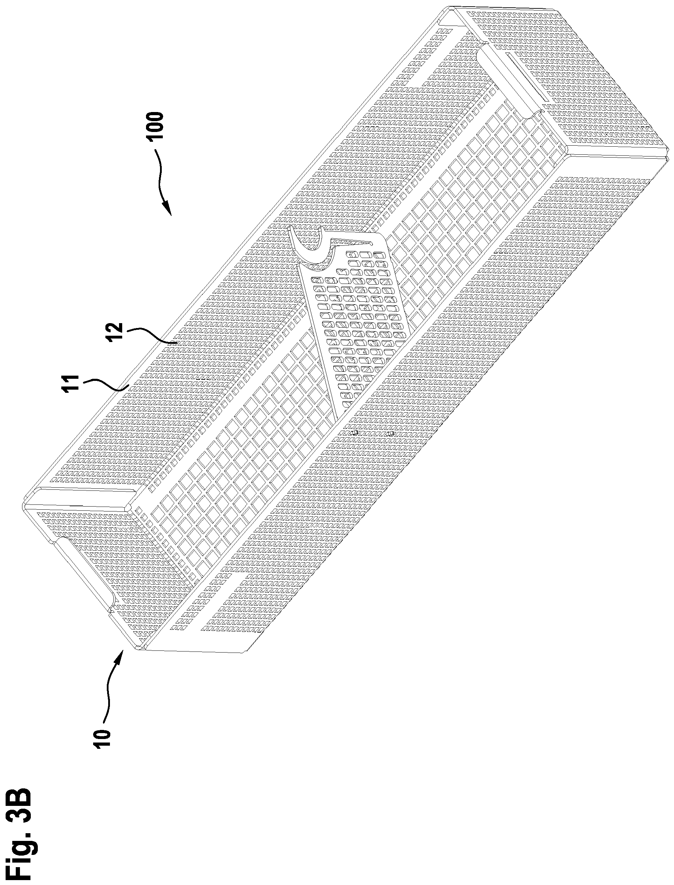

In den Fig. 3A bis 4B ist gezeigt, wie dieses Trennelement 100 in einen Siebkorb 10 eingesetzt werden kann. Zunächst wird das Trennelement 100 in den Siebkorb 10 eingeführt und die L-förmigen Vorsprünge 121 werden in zwei übereinander angeordnete Löcher bzw. Aussparungen 12 der Perforation des Siebkorbs eingeführt. Anschließend wird das Trennelement 100 um die L-förmigen Vorsprünge 121 gedreht. Dabei wird der federelastische Abschnitt 112 mit Hilfe des Eingriffsabschnitts 113 zu dem zentralen Wandabschnitt 101 hin verformt, um die laterale Ausdehnung des Trennelements vorübergehend zu verringern, sodass die Vorsprünge 114 nicht in die Löcher des Siebkorbes eingreifen, bis das Trennelement 100 die gewünschte Position erreicht hat. Die freien Enden der L-förmigen Vorsprünge 121 umgreifen dabei einen der Stege, welcher zwischen benachbarten Aussparungen der Perforation12 des Siebkorbes ausgebildet sind. Wenn das Trennelement 100 die gewünschte Position erreicht hat, welche in der Regel exakt senkrecht zu den beiden Wänden des Siebkorbs 10 ist. Wird der federelastische Abschnitt 112 losgelassen und kehrt in seine Ausgangsposition bzw. -form zurück, wobei die Vorsprünge 114 in zwei Aussparungen 12 der Wand des Siebkorbs 10 eingreifen. Während des Verschwenkens des Trennelements 100 um die L-förmigen Vorsprünge 121 gleitet der Anlagevorsprung 103 über den Boden des Siebkorbs und dient so zur korrekten vertikalen Ausrichtung des Trennelements 100.

Die Löcher 102 und die Vorsprünge 114 und 121 können auch derart angeordnet sein, dass ein Trennelement 100 auch auf einer Seite oder sogar beiden Seiten in einem gleichartigen Trennelement 100 verankert werden können. Dabei können die Trennelemente 100 unterschiedliche Längen und/oder Höhen aufweisen, um auf diese Weise eine sehr individuelle Unterteilung eines Siebkorbs zu ermöglichen.

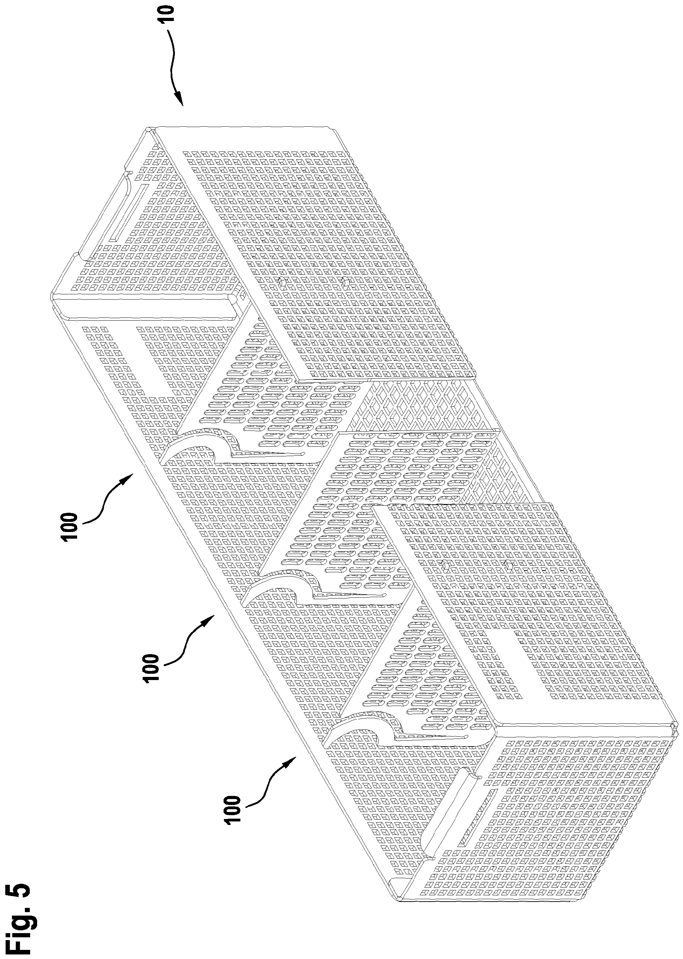

In der Fig. 5 ist ein Siebkorb 10 gezeigt, in den drei Trennelemente 100 des ersten Ausführungsbeispiels eingesetzt sind. In der Ansicht der Fig. 5 ist zur besseren Veranschaulichung ein Teil einer Seitenwand des Siebkorbes 10 ausgespart.

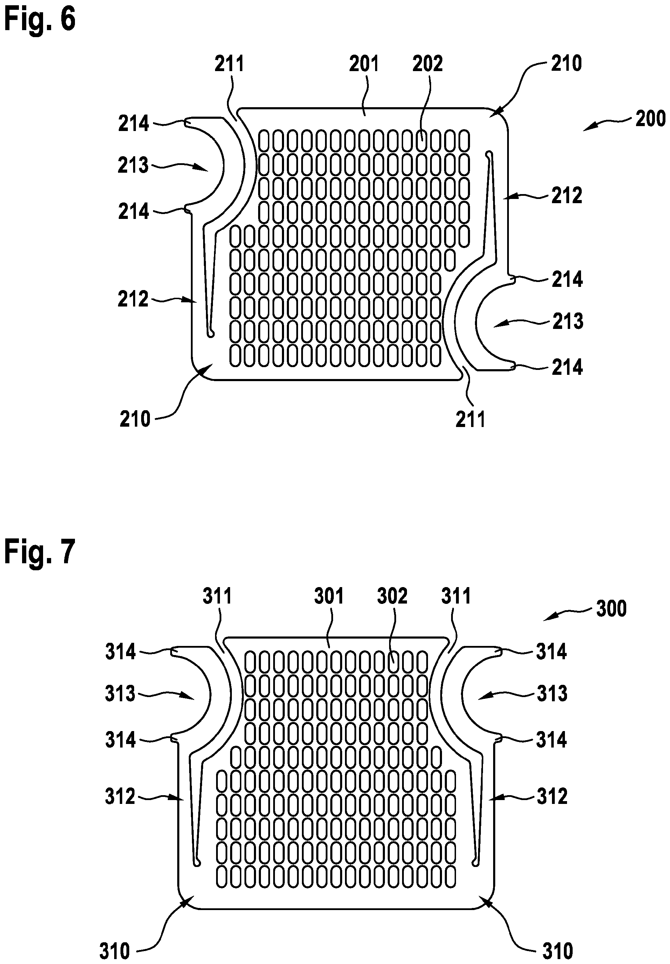

Ein zweites Ausführungsbeispiel der vorliegenden Erfindung ist in Fig. 6 gezeigt. Ein Trennelement 200 weist einen zentralen Wandabschnitt 201 mit Löchern 202 und zwei laterale Wandabschnitte 210 auf. In jedem dieser Wandabschnitte 210 ist eine schlitzförmige Aussparung 211 ausgebildet, welche einen federelastischen Abschnitt 212 von dem zentralen Wandabschnitt 201 separiert. An jedem federelastischen Abschnitt 112 sind zwei Vorsprünge 214 ausgebildet, zwischen denen jeweils ein Eingriffsabschnitt 213 in Form einer bogenförmigen bzw. kreisbogenförmigen Aussparung ausgebildet ist. Während sich eine der beiden schlitzförmigen Aussparungen 211 von oben in das Trennelement 200 erstreckt, erstreckt sich die andere schlitzförmige Aussparung 211 von unten in das Trennelement 200. Auf diese Weise ist ein rotationssymmetrisches Trennelement 200 ausgebildet, bei welchem es keine eindeutige Oberseite bzw. Unterseite mehr gibt. Das Trennelement 200 ist aus einem Metall additiv gefertigt.

Ein solches Trennelement 200 wird in einen Siebkorb 10 bevorzugt so eingesetzt, dass zunächst diejenigen Vorsprünge 114 eines der beiden lateralen Wandabschnitte 210 in einer Wand des Siebkorbs 10 verankert werden, welche sich insgesamt näher zu dem Boden des Siebkorbs 10 befinden. Anschließend wird das Trennelement 200 um diese Vorsprünge herum gedreht bzw. geschwenkt und der Einsetzvorgang geht weiter wie beim ersten Ausführungsbeispiel. Es ist aber auch möglich, zunächst diejenigen Vorsprünge 114 eines Wandabschnitts 210 in einer Wand eines Siebkorbs 10 zu verankern, welche sich insgesamt näher zu dem oberen Rand des Siebkorbs 10 befinden.

Die Vorsprünge 114 beider lateralen Wandabschnitte 210 können aber auch auf jeweils gleicher Höhe angeordnet sein, sodass es dann keine Rolle spielt, welche Vorsprünge zuerst in welcher Wand des Siebkorbs 10 oder eines anderen Trennelements 100 oder 200 verankert werden. Es ist auch möglich, dass beide federelastischen Abschnitte 212 verformt werden und so alle Vorsprünge 114 gleichzeitig in der jeweiligen Wand des Siebkorbs 10 oder in einem anderen Trennelement 100, 200 verankert werden, indem sie in die Aussparungen 12 derselben eingeführt werden.

In der Fig. 7 ist ein drittes Ausführungsbeispiel der vorliegenden Erfindung gezeigt. Dieses Trennelement 300 weist ebenfalls einen zentralen Wandabschnitt 301 mit Löchern 302 und zwei laterale Wandabschnitte 310 auf. In jedem lateralen Wandabschnitt 310 ist wiederum eine schlitzförmige Aussparung 311 vorgesehen, welche einen federelastischen Abschnitt 312 ausbildet, an dem wiederum je zwei Vorsprünge 314 vorgesehen sind, zwischen welchen jeweils ein Eingriffsabschnitt 313 ausgebildet ist. Im Unterschied zum zweiten Ausführungsbeispiel erstrecken sich die beiden schlitzförmigen Aussparungen 311 von derselben Seite in das Trennelement 300, sodass dieses spiegelsymmetrisch ausgebildet ist. Das Einsetzen dieses Trennelements 300 geschieht im Wesentlichen in derselben Weise, wie es bei dem zweiten Ausführungsbeispiel möglich ist. Das dritte Ausführungsbeispiel ist aus einem festen Kunststoff gefertigt.

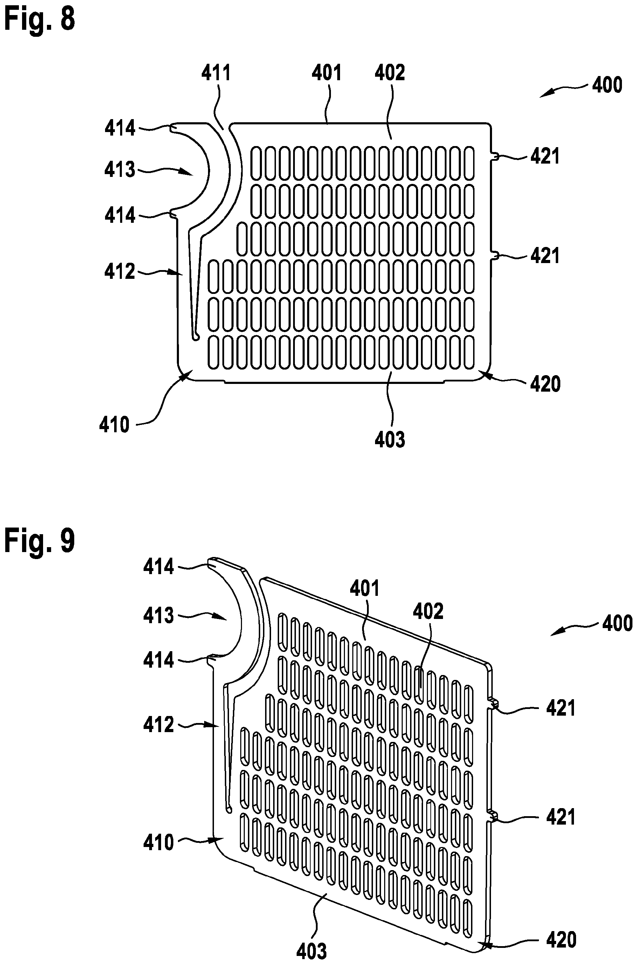

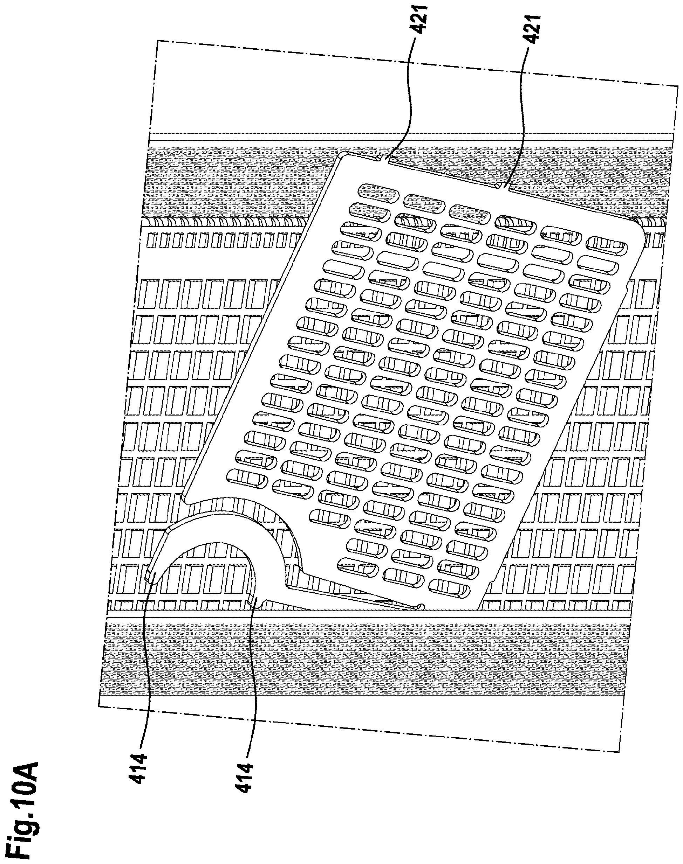

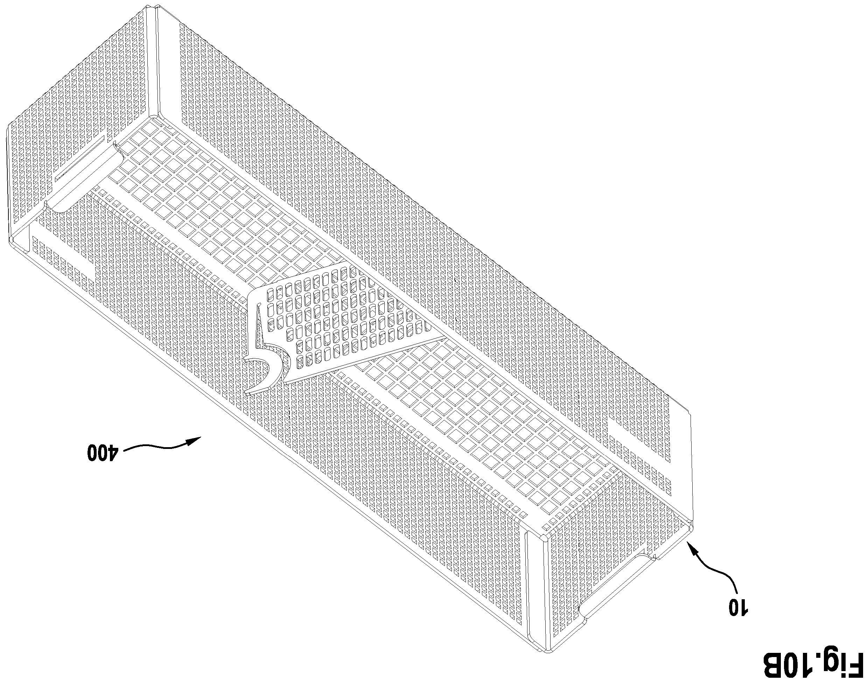

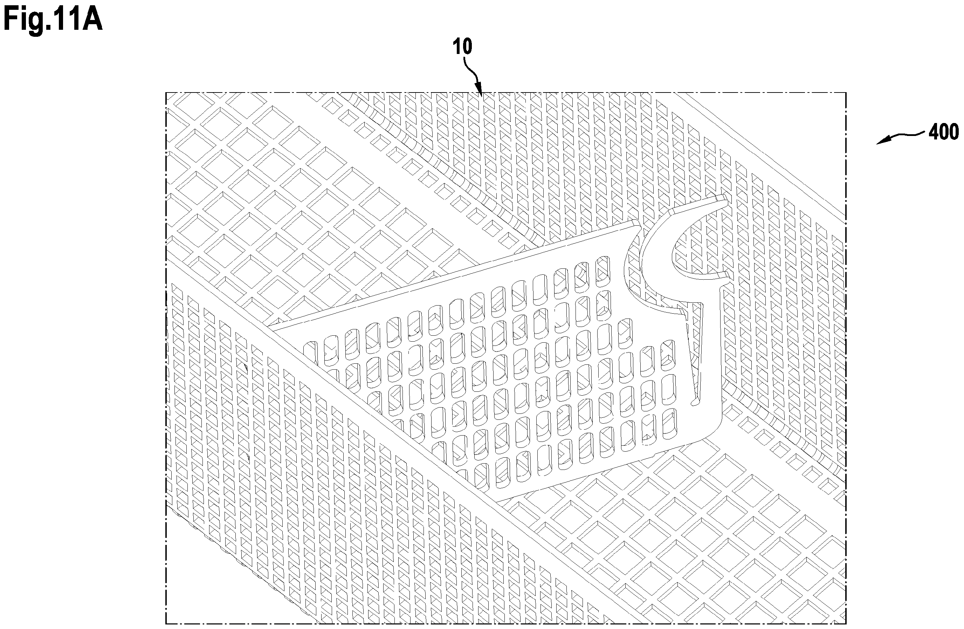

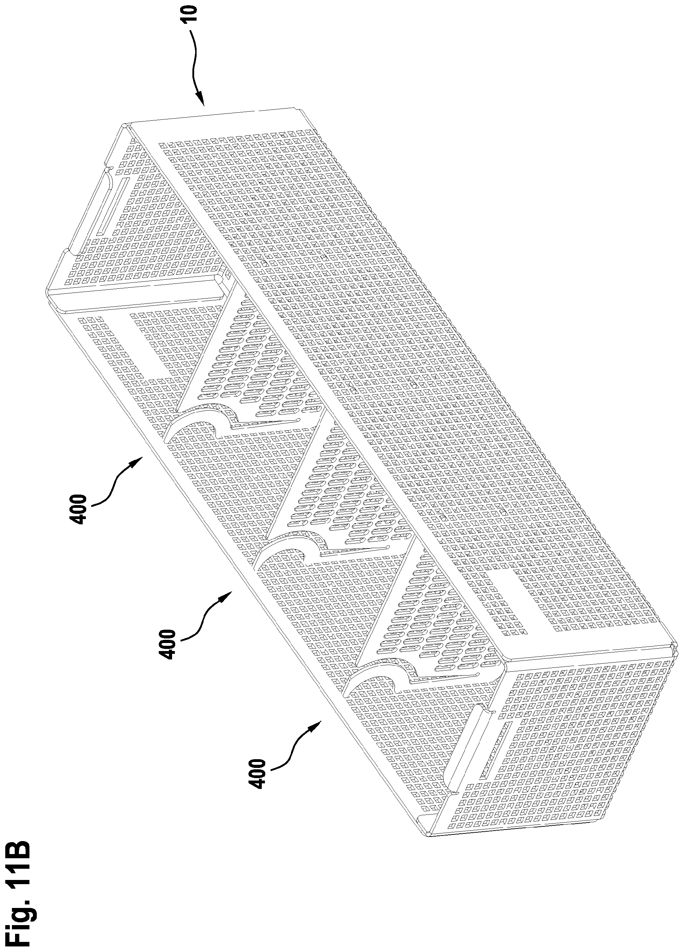

In den Fig. 8 bis 11 ist ein viertes Ausführungsbespiel der vorliegenden Erfindung gezeigt. Das Trennelement 400 dieses vierten Ausführungsbeispiels unterscheidet sich von dem Trennelement 100 des ersten Ausführungsbeispiels lediglich dadurch, dass die Vorsprünge 421 als laterale Vorsprünge ausgebildet sind, wohingegen die Vorsprünge 121 des ersten Ausführungsbeispiels als L-förmige Vorsprünge ausgebildet sind. Die Vorsprünge 421 de vierten Ausführungsbeispiels erstrecken sich vollständig in der Ebene der Trennwand.

Dem Fachmann ergeben sich zahlreiche Abwandlungen und Modifikationen aus den verschiedenen Ausführungsbeispielen. Die einzelnen Merkmale der Ausführungsbeispiele können vielfach sinnvoll miteinander kombiniert werden. Beispielsweise kann ein Anlagevorsprung 103 auch an der Unterseite des Trennelements 300 ausgebildet sein oder an der Oberseite und/oder Unterseite des Trennelements 200. Die Materialien können ebenfalls beliebig miteinander vertauscht werden.

Die schlitzförmigen Aussparungen 111, 211, 311 sind nicht auf eine bestimmte Form beschränkt und schlitzartig bedeutet hierbei auch nicht, dass die Aussparung besonders schmal sein muss. Die Aussparung muss eine hinreichende elastische Verformbarkeit des federelastischen Abschnitts 112, 212, 312 ermöglichen und sollte gleichzeitig nicht so groß sein, dass das Trennelement 100, 200, 300 den Siebkorb 10 nicht mehr wirkungsvoll unterteilen kann. Dies hängt auch von der Größe der Elemente ab, welche in dem Siebkorb gelagert werden sollen.

### Bezugszeichenliste

- 10: Siebkorb
- 11: Seitenwand
- 12: Aussparung, Perforation
- 100: Trennelement
- 101: zentraler Wandabschnitt
- 102: Aussparung, Perforation
- 103: Anlagevorsprung
- 110: lateraler Bereich
- 111: Aussparung
- 112: federelastischer Abschnitt
- 113: Eingriffsabschnitt
- 114: laterale Vorsprünge
- 120: lateraler Bereich
- 121: L-förmige Vorsprünge
- 200: Trennelement
- 201: zentraler Wandabschnitt
- 202: Aussparung, Perforation
- 210: lateraler Bereich
- 211: Aussparung
- 212: federelastischer Abschnitt
- 213: Eingriffsabschnitt
- 214: laterale Vorsprünge
- 300: Trennelement
- 301: zentraler Wandabschnitt
- 302: Aussparung, Perforation
- 310: lateraler Bereich
- 311: Aussparung
- 312: federelastischer Abschnitt
- 313: Eingriffsabschnitt
- 314: laterale Vorsprünge
- 400: Trennelement
- 401: zentraler Wandabschnitt
- 402: Aussparung, Perforation
- 403: Anlagevorsprung
- 410: lateraler Bereich
- 411: Aussparung
- 412: federelastischer Abschnitt
- 413: Eingriffsabschnitt
- 414: laterale Vorsprünge
- 420: lateraler Bereich
- 421: laterale Vorsprünge

## Patentansprüche

1. Trennelement (100, 200, 300, 400) für einen Sterilisationssiebkorb (10) zur Sterilisation von medizinischen Instrumenten mit
zwei lateralen Wandabschnitten und einem dazwischen liegenden zentralen Wandabschnitt (101, 201, 301, 401), wobei
das Trennelement (100, 200, 300, 400) im Wesentlichen flächig ausgebildet ist, und
der zentrale Wandabschnitt (101, 201, 301, 401) mit einer Vielzahl von Durchbrechungen (102, 202, 302, 402) ausgebildet ist,
**dadurch gekennzeichnet, dass**
der eine laterale Wandabschnitt eine schlitzförmige Aussparung (111, 211, 311, 411) aufweist, sodass der durch die schlitzförmige Aussparung (111, 211, 311, 411) separierte, laterale Bereich (110, 210, 310, 410) dieses lateralen Wandabschnitts als federelastischer Abschnitt (112, 212, 312, 412) ausgebildet ist, wobei der federelastische Abschnitt (112, 212, 312, 412) eine Anzahl von Vorsprüngen (114, 214, 314, 414) aufweist, welche sich zumindest in lateraler Richtung erstrecken,
wobei der federelastische Abschnitt (112, 212, 312, 412) so elastisch zu dem zentralen Wandabschnitt (101, 201, 301, 401) hin verformbar ist, dass sich die maximale laterale Erstreckung des Trennelements (100, 200, 300, 400) verringert, und
der andere laterale Wandabschnitt eine Anzahl von Vorsprüngen (121, 214, 314, 421) aufweist, welche sich zumindest teilweise in lateraler Richtung erstrecken,
wobei die Anzahl von Vorsprüngen (114, 121, 214, 314, 414, 421) daran angepasst ist, in einer Wand eines Sterilisationssiebkorbes (10) und/oder in einem anderen Trennelement (100, 200, 300, 400) verankert zu werden.

2. Trennelement (100, 200, 300, 400) für einen Sterilisationssiebkorb (10) zur Sterilisation von medizinischen Instrumenten nach Anspruch 1, wobei
sich wenigstens ein Vorsprung (121) des anderen lateralen Wandabschnitts zumindest teilweise in eine Richtung senkrecht zur Erstreckungsebene des zentralen Wandabschnitts (101, 201, 301, 401) erstreckt.

3. Trennelement (100, 200, 300, 400) für einen Sterilisationssiebkorb (10) zur Sterilisation von medizinischen Instrumenten nach Anspruch 2, wobei
die Vorsprünge (121) des anderen lateralen Wandbereichs im Wesentlichen L-förmig ausgebildet sind und sich von dem anderen lateralen Wandabschnitt zunächst in lateraler Richtung erstrecken und im weiteren Verlauf in einer Richtung senkrecht zur Fläche des zentralen Wandabschnitts(101, 201, 301, 401).

4. Trennelement (100, 200, 300, 400) für einen Sterilisationssiebkorb (10) zur Sterilisation von medizinischen Instrumenten nach Anspruch 1,
**dadurch gekennzeichnet, dass**
der andere laterale Wandabschnitt eine schlitzförmige Aussparung (211, 311) aufweist, sodass der durch die schlitzförmige Aussparung (211, 311) separierte, laterale Bereich (210, 310) des anderen lateralen Wandabschnitts als federelastischer Abschnitt (212, 312) ausgebildet ist.

5. Trennelement (100, 200, 300, 400) für einen Sterilisationssiebkorb (10) zur Sterilisation von medizinischen Instrumenten nach Anspruch 4, wobei
das Trennelement (100, 200, 300, 400) spiegelsymmetrisch ausgebildet ist.

6. Trennelement (100, 200, 300, 400) für einen Sterilisationssiebkorb (10) zur Sterilisation von medizinischen Instrumenten nach Anspruch 4, wobei
das Trennelement (100, 200, 300, 400) rotationssymmetrisch ausgebildet ist.

7. Trennelement (100, 200, 300, 400) für einen Sterilisationssiebkorb (10) zur Sterilisation von medizinischen Instrumenten nach einem der Ansprüche 1 bis 6, wobei
jeder laterale Wandbereich wenigstens zwei Vorsprünge (114, 214, 314, 414) aufweist.

8. Trennelement (100, 200, 300, 400) für einen Sterilisationssiebkorb (10) zur Sterilisation von medizinischen Instrumenten nach einem der Ansprüche 1 bis 7, wobei
an einem federelastischen Abschnitt (12, 212, 312, 412) ein Eingriffsabschnitt (113, 213, 313, 413) ausgebildet ist.

9. Trennelement (100, 200, 300, 400) für einen Sterilisationssiebkorb (10) zur Sterilisation von medizinischen Instrumenten nach Anspruch 8, wobei
der Eingriffsabschnitt (113, 213, 313, 413) zwischen zwei Vorsprüngen (114, 214, 314, 414) ausgebildet ist.

10. Trennelement (100, 200, 300, 400) für einen Sterilisationssiebkorb (10) zur Sterilisation von medizinischen Instrumenten nach Anspruch 8 oder 9, wobei
der Eingriffsabschnitt (113, 213, 313, 413) als laterale Aussparung ausgebildet ist.

11. Trennelement (100, 200, 300, 400) für einen Sterilisationssiebkorb (10) zur Sterilisation von medizinischen Instrumenten nach einem der Ansprüche 1 bis 10, wobei
das Trennelement (100, 200, 300, 400) aus Metall ausgebildet ist, vorzugsweise aus einem Metallblech.

## Claims

1. Separating element (100, 200, 300, 400) for a perforated sterilization basket (10) for sterilizing medical instruments, comprising
two lateral wall portions and a central wall portion (101, 201, 301, 401) located therebetween, wherein
the separating element (100, 200, 300, 400) is substantially planar, and
the central wall portion (101, 201, 301, 401) has a plurality of throughopenings (102, 202, 302, 402),
**characterized in that**
one lateral wall portion has a slot-shaped cut-out (111, 211, 311, 411), and so the lateral region (110, 210, 310, 410) of this lateral wall portion separated by the slot-shaped cut-out (111, 211, 311, 411) is in the form of a resilient portion (112, 212, 312, 412), wherein the resilient portion (112, 212, 312, 412) has a number of projections (114, 214, 314, 414) which extend at least in the lateral direction,
wherein the resilient portion (112, 212, 312, 412) is elastically deformable toward the central wall portion (101, 201, 301, 401) to such an extent that the maximum lateral extension of the separating element (100, 200, 300, 400) is reduced, and
the other lateral wall portion has a number of projections (121, 214, 314, 421) which extend at least partially in the lateral direction,
wherein the number of projections (114, 121, 214, 314, 414, 421) is adapted to be anchored in a wall of a perforated sterilization basket (10) and/or in another separating element (100, 200, 300, 400).

2. Separating element (100, 200, 300, 400) for a perforated sterilization basket (10) for sterilizing medical instruments according to claim 1, wherein
at least one projection (121) of the other lateral wall portion extends at least partially in a direction perpendicular to the extension plane of the central wall portion (101, 201, 301, 401).

3. Separating element (100, 200, 300, 400) for a perforated sterilization basket (10) for sterilizing medical instruments according to claim 2, wherein
the projections (121) of the other lateral wall region are substantially L-shaped and initially extend in the lateral direction from the other lateral wall portion and, as they progress, extend in a direction perpendicular to the surface of the central wall portion (101, 201, 301, 401).

4. Separating element (100, 200, 300, 400) for a perforated sterilization basket (10) for sterilizing medical instruments according to claim 1,
**characterized in that**
the other lateral wall portion has a slot-shaped cut-out (211, 311), and so the lateral region (210, 310) of the other lateral wall portion separated by the slot-shaped cut-out (211, 311) is in the form of a resilient portion (212, 312).

5. Separating element (100, 200, 300, 400) for a perforated sterilization basket (10) for sterilizing medical instruments according to claim 4, wherein
the separating element (100, 200, 300, 400) is mirror-symmetrical.

6. Separating element (100, 200, 300, 400) for a perforated sterilization basket (10) for sterilizing medical instruments according to claim 4, wherein
the separating element (100, 200, 300, 400) is rotationally symmetrical.

7. Separating element (100, 200, 300, 400) for a perforated sterilization basket (10) for sterilizing medical instruments according to any of claims 1 to 6, wherein
each lateral wall region has at least two projections (114, 214, 314, 414).

8. Separating element (100, 200, 300, 400) for a perforated sterilization basket (10) for sterilizing medical instruments according to any of claims 1 to 7, wherein
an engagement portion (113, 213, 313, 413) is formed on a resilient portion (12, 212, 312, 412).

9. Separating element (100, 200, 300, 400) for a perforated sterilization basket (10) for sterilizing medical instruments according to claim 8, wherein
the engagement portion (113, 213, 313, 413) is formed between two projections (114, 214, 314, 414).

10. Separating element (100, 200, 300, 400) for a perforated sterilization basket (10) for sterilizing medical instruments according to claim 8 or 9, wherein
the engagement portion (113, 213, 313, 413) is in the form of a lateral recess.

11. Separating element (100, 200, 300, 400) for a perforated sterilization basket (10) for sterilizing medical instruments according to any of claims 1 to 10, wherein
the separating element (100, 200, 300, 400) is formed from metal, preferably from a metal sheet.

## Revendications

1. Élément de séparation (100, 200, 300, 400) pour un panier perforé de stérilisation (10) destiné à la stérilisation d'instruments médicaux, comportant
deux sections de paroi latérales et une section de paroi centrale (101, 201, 301, 401) située entre elles, dans lequel
l'élément de séparation (100, 200, 300, 400) est réalisé sensiblement de manière plane, et
la section de paroi centrale (101, 201, 301, 401) est conçue avec une pluralité de perforations (102, 202, 302, 402),
**caractérisé en ce que**
l'une des sections de paroi latérales présente un évidement en forme de fente (111, 211, 311, 411), de manière à ce que la zone latérale (110, 210, 310, 410) de cette section de paroi latérale, séparée par l'évidement en forme de fente (111, 211, 311, 411), soit réalisée sous la forme de section élastique (112, 212, 312, 412), dans lequel la section élastique (112, 212, 312, 412) présente un certain nombre de saillies (114, 214, 314, 414) qui s'étendent au moins dans le sens latéral,
dans lequel la section élastique (112, 212, 312, 412) est déformable de manière élastique vers la section de paroi centrale (101, 201, 301, 401), de telle sorte que l'extension latérale maximale de l'élément de séparation (100, 200, 300, 400) diminue, et
l'autre section de paroi latérale présente un certain nombre de saillies (121, 214, 314, 421) qui s'étendent au moins partiellement dans le sens latéral,
dans lequel le nombre de saillies (114, 121, 214, 314, 414, 421) est adapté pour être ancré dans une paroi d'un panier perforé de stérilisation (10) et/ou dans un autre élément de séparation (100, 200, 300, 400).

2. Élément de séparation (100, 200, 300, 400) pour un panier perforé de stérilisation (10) destiné à la stérilisation d'instruments médicaux selon la revendication 1, dans lequel
au moins une saillie (121) de l'autre section de paroi latérale s'étend au moins partiellement dans un sens perpendiculaire au plan d'extension de la section de paroi centrale (101, 201, 301, 401).

3. Élément de séparation (100, 200, 300, 400) pour un panier perforé de stérilisation (10) destiné à la stérilisation d'instruments médicaux selon la revendication 2, dans lequel
les saillies (121) de l'autre zone de paroi latérale sont réalisées sensiblement en forme de L et s'étendent d'abord dans le sens latéral à partir de l'autre section de paroi latérale et par la suite dans un sens perpendiculaire à la surface de la section de paroi centrale (101, 201, 301, 401).

4. Élément de séparation (100, 200, 300, 400) pour un panier perforé de stérilisation (10) destiné à la stérilisation d'instruments médicaux selon la revendication 1,
**caractérisé en ce que**
l'autre section de paroi latérale présente un évidement en forme de fente (211, 311), de manière à ce que la zone latérale (210, 310) de l'autre section de paroi latérale, séparée par l'évidement en forme de fente (211, 311), soit réalisée sous la forme de section élastique (212, 312).

5. Élément de séparation (100, 200, 300, 400) pour un panier perforé de stérilisation (10) destiné à la stérilisation d'instruments médicaux selon la revendication 4, dans lequel
l'élément de séparation (100, 200, 300, 400) est réalisé avec une symétrie miroir.

6. Élément de séparation (100, 200, 300, 400) pour un panier perforé de stérilisation (10) destiné à la stérilisation d'instruments médicaux selon la revendication 4, dans lequel
l'élément de séparation (100, 200, 300, 400) est réalisé de manière symétrique en rotation.

7. Élément de séparation (100, 200, 300, 400) pour un panier perforé de stérilisation (10) destiné à la stérilisation d'instruments médicaux selon l'une des revendications 1 à 6, dans lequel
chaque zone de paroi latérale présente au moins deux saillies (114, 214, 314, 414).

8. Élément de séparation (100, 200, 300, 400) pour un panier perforé de stérilisation (10) destiné à la stérilisation d'instruments médicaux selon l'une des revendications 1 à 7, dans lequel
une section de prise (113, 213, 313, 413) est réalisée sur une section élastique (12, 212, 312, 412).

9. Élément de séparation (100, 200, 300, 400) pour un panier perforé de stérilisation (10) destiné à la stérilisation d'instruments médicaux selon la revendication 8, dans lequel
la section de prise (113, 213, 313, 413) est réalisée entre deux saillies (114, 214, 314, 414).

10. Élément de séparation (100, 200, 300, 400) pour un panier perforé de stérilisation (10) destiné à la stérilisation d'instruments médicaux selon la revendication 8 ou 9, dans lequel
la section de prise (113, 213, 313, 413) est réalisée sous la forme d'un évidement latéral.

11. Élément de séparation (100, 200, 300, 400) pour un panier perforé de stérilisation (10) destiné à la stérilisation d'instruments médicaux selon l'une des revendications 1 à 10, dans lequel
l'élément de séparation (100, 200, 300, 400) est réalisé en métal, de préférence à partir d'une tôle métallique.
